# EUROPEAN PATENT APPLICATION

(11) **EP 4 311 539 A1**
(43) Date of publication of application: **31.01.2024**
(21) Application number: 22187889.5
(22) Date of filing: 29.07.2022
(51) Int. Cl.: A61K 9/00, A61K 9/19, A61K 31/00, A61K 47/02, A61K 47/20, A61K 47/26, A61P 7/02, A61P 9/10

(54) **IMATINIB FORMULATION FOR PARENTERAL ADMINISTRATION**

(71) Applicant: Artorange Ltd., 3083 Limassol (CY)
(72) Inventor: Rolava, Evija, 2114 Olaine, Olaine district (LV); Pitkevica, Olga, 2114 Olaine, Olaine distric (LV); Skrivelis, Vitalijs, 2114 Olaine, Olaine distric (LV); Krauklis, Vjaceslavs, 3083 Limassol (CY); Arsenian, Sergey, 3083 Limassol (CY)
(74) Representative: Ullrich & Naumann PartG mbB

(57) **Abstract**

The application relates to a pharmaceutical composition comprising Imatinib and a stabilizing formulation, wherein said stabilizing formulation comprises a bulking agent and a buffering agent, wherein the pH of the pharmaceutical composition is below 6.0. Further, the application relates to a ready-to-use solution for use in the treatment of stroke, traumatic brain injury or spinal cord injuries, which is reconstituted from a lyophilized pharmaceutical composition by the addition of an aqueous diluent. Moreover, the application relates to a method for preparing the pharmaceutical composition.

## Description

### FIELD OF THE INVENTION

The present invention relates to pharmaceutical compositions of Imatinib for use in parenteral administration for the treatment of stroke, traumatic brain injury or spinal cord injuries as well the preparation of such pharmaceutical compositions.

### BACKGROUND OF THE INVENTION

A stroke is defined as the sudden death of brain cells in a localized area due to interrupted or inadequate blood flow. According to the European Cardiovascular Disease Statistics 2012, stroke is the second single most common cause of death in Europe: accounting for almost 1.1 million deaths each year. Over one in seven women (15%) and one in ten men (10%) die from the disease (Melanie Nichols et al, European Cardiovascular Disease Statistics, 2012 Edition, Department of Public Health, University of Oxford).

Depending on the causative factor and progression, a stroke can be designated as one of the following two main types: Ischemic stroke, which occurs as a result of an obstruction, or clot, within a blood vessel supplying blood to the brain. This accounts for the vast majority of all stroke cases.

Hemorrhagic stroke, which occurs when a blood vessel ruptures. The reason for the rupture can be an aneurysm or an arteriovenous malformation, which can be congenital defects. The most common cause is however uncontrolled hypertension. The following disclosure will focus on ischemic stroke, and in particular acute ischemic stroke, but is not limited thereto. The goal of all existing treatments of ischemic stroke is to quickly restore the blood flow and to prevent or at least reduce the likelihood of another stroke. Currently, the only FDA approved treatment for ischemic strokes is tissue plasminogen activator (tPA, ALTEPLASE^{®}, ACTIVASE^{®}). tPA works by dissolving the clot and improving blood flow to the part of the brain being deprived of blood flow. If administered early tPA may improve the chances of recovering from a stroke. tPA is however known to have serious side effects such as neurotoxicity and increased vascular permeability due to an altered blood-brain barrier function. There are indications that the balance between the positive effects of tPA and the side effects becomes less favorable to the patient when the onset of tPA therapy is delayed. The exact thresholds vary, but it is recommended in general that tPA therapy should be started as soon as possible, and preferably not later than 4-5 hours after the stroke. Sometimes an even narrower therapeutic window is recommended.

Before administering tPA or other pharmaceutical thrombolysis, it has to be determined if the patient suffers from an acute ischemic stroke of from a hemorrhagic stroke. In the case of haemorrhage, the thrombolysis would worsen the condition. The symptoms alone are usually not specific enough to make this distinction, so usually the patient is subjected to a CT scan or MRI scan to rule out any intracranial haemorrhage. Only then can thrombolytic treatment be started. Depending on the available resources and diagnostic capabilities, this may delay the starting time of thrombolytic therapy, or even make the care giver refrain from such therapy altogether as a precaution. Further, in order to be most effective, thrombolysis should be started within 3 hours from the occurrence of the stroke, preferably as early as possible.

Thrombolysis is the treatment of choice for acute stroke within 3 to 4.5 hours after symptom onset. There are trials indicating that alteplase 3-4.5 h after onset was better than placebo, and a registry study supporting this.

Professional recommendations have issued in favour of these results but remain to be fully accepted. However, there are indications that late onset thrombolytic therapy may be associated with more severe side effects. As a consequence, it is possible that a patient does not receive the most appropriate therapy, either because of an uncertainty in the diagnosis (ischemic v. hemorrhagic stroke), or a delay in reaching the diagnosis (delays in obtaining a CT, or the lack of the necessary equipment), or a combination. In practice, only about 10 % of the patients receive thrombolytic therapy.

Other treatments include mechanical thrombectomy, i.e. the surgical removal or disintegration of the clot, and antiplatelet therapy, preventing new clots from forming. Cumulating experience shows that thrombectomy significantly increases the odds of recovery or at least reduces the neurological damage following a stroke. Unlike thrombolytic drugs, the anti-platelet agents do not have effect on clots that are already present. Aspirin is the most commonly used antiplatelet agent, and there are indications that it is effective for the early treatment of acute ischemic stroke, preferably in combination with dipyridamol.

WO 2007/124308 discloses methods and compositions for preventing or ameliorating the side effects, and for improving the treatment, or extending the treatment window, of tPA where thrombolysis is desired, by using platelet-derived growth factor (PDGF) and PDGF receptor antagonism to counter the undesirable effects of tPA. WO 2007/124308 also discloses a combination therapy in the treatment of ischemic stroke using tPA and a PDGF receptor antagonist. Imatinib mesylate is given as one example of suitable substances that inhibit the PDGF receptor.

WO 2017/151043 A1 describes that Imatinib (GLEEVEC^{®}, GLIVEC^{®}, STI-571, a tyrosine kinase inhibitor) can be used in the treatment of acute ischemic stroke, wherein said Imatinib is administered to a patient at a dose of 650 mg/day or higher for at least 3 consecutive days

A traumatic brain injury (TBI) is a disruption of the normal function of the brain that can be caused by a bump, blow or jolt to the head or a penetrating head injury. While everyone is at risk for a TBI, children and older adults are especially vulnerable. Spinal cord injuries (SCI) describe injuries to the spinal cord. Symptoms of SCI can include partial or complete loss of sensory function or motor control of the arms, legs or body. In severe cases, SCI can affect bladder and bowel control, breathing, heart rate and blood pressure.

Su et al. 2015 examined the efficacy for the treatment of TBI of Imatinib. Using a murine model the authors showed that Imatinib treatment significantly reduces blood brain barrier (BBB) dysfunction. This is associated with significantly reduced lesion size reduced cerebral edema, and with the preservation of cognitive function. Accordingly, data suggests the treatment of TBI with Imatinib.

Abrams et al. 2012 investigated whether Imatinib (GleevecH, Novartis) could improve functional outcome in experimental SCI. Rats subjected to contusion spinal cord injury were treated orally with Imatinib for 5 days beginning 30 minutes after injury. The authors found that Imatinib significantly enhanced blood-spinal cord-barrier integrity, hindlimb locomotor function, sensorimotor integration, and bladder function, as well as attenuated astrogliosis and deposition of chondroitin sulfate proteoglycans, and increased tissue preservation. These improvements were associated with enhanced vascular integrity and reduced inflammation. The positive effects on experimental SCI, makes Imatinib an interesting candidate drug for clinical trials in SCI.

To date, Imatinib has been only made available as pharmaceutical forms for oral administration. However, WO 2017/151043 A1 suggests also other administration forms, i.e. intravenous administration. Thus, it is an object of the invention to provide a formulation of Imitainib for parenteral administration.

### SUMMARY OF THE INVENTION

The present inventors have identified a formulation for Imatinib, which is suitable for parenteral administration, displays high solubility and an excellent chemical and physical stability over the shelf-life of the product. Accordingly, in a first aspect, the invention provides a pharmaceutical composition comprising Imatinib and a stabilizing formulation, wherein said stabilizing formulation comprises a bulking agent and a buffering agent, wherein the pH of the pharmaceutical composition is below 6.0.

The pharmaceutical composition according to the first aspect is preferably stored in a lyophilized form and reconstituted into a ready-to-use solution for the treatment of stroke. Thus, in a second aspect, the invention relates to a ready-to-use solution for use in the treatment of stroke, which is reconstituted from a lyophilized pharmaceutical composition according to the first aspect by the addition of an aqueous diluent.

Moreover, the present invention provides a method for preparing a pharmaceutical composition according to the first aspect comprising the steps of:
i) providing a first container with a first part of the solvent, in particular 40 % to 90 % of the volume;
ii) adding to the first part of the solvent the buffering agent and the bulking agent and mixing to form an intermediate solution;
iii) adding the Imatinib to the first container and mixing the Imatinib with the intermediate solution to obtain a homogenous Imatinib suspension;
iv) providing a second container with a second part of the solvent, in particular 10 % to 50 % of the volume;
v) adding the antioxidant to the second part of the solvent in the second container, mixing and heating the mixture to obtain an antioxidant solution;
vi) adding the antioxidant solution to the Imatinib suspension, optionally adding further solvent, and mixing to form the pharmaceutical composition; and
vii) optionally adjusting the pH.

### DETAILED DESCRIPTION OF THE INVENTION

The present invention provides novel formulations of Imatinib for parenteral administration. In the pharmaceutical composition, the Imatinib is highly stable and readily transferable to a lyophilized state. The pharmaceutical composition when in liquid form is essentially free from any visible particulates. Moreover, the components of the formulation are well tolerated by the human body when administered parenterally.

Accordingly, in a first aspect, the invention provides a pharmaceutical composition comprising Imatinib and a stabilizing formulation, wherein said stabilizing formulation comprises a bulking agent and a buffering agent, wherein the pH of the pharmaceutical composition is below 6.0.

Imatinib mesilate chemical name is 4-[(4-methylpiperazin-1-yl)methyl]-N-[4-methyl-3-[(4-pyridin-3-ylpyrimidin-2-yl)amino]phenyl]benzamide methanesulfonate. CAS No: 220127-57-1.

It is white or almost white, slightly brownish or yellowish, or brownish yellow powder. The molecular formula is C29H31N7O • CH4O3S and molecular weight is 589.70 g/mol. The molecular weight of Imatinib is 493,603 g/mol.

The structural formula is:

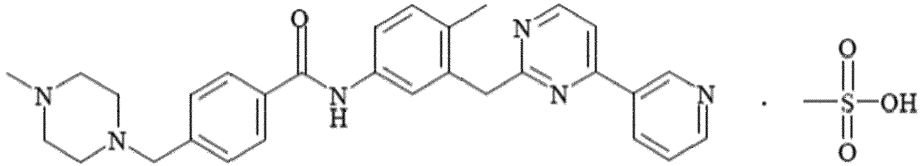

The bulking agent provides the structure of the freeze-dried product without interacting directly with the pharmaceutical product and enhances the stability over long-term storage. In addition, the bulking agent may impart further useful qualities such as modifying the collapse temperature or providing freeze-thaw protection. Non-limiting examples of bulking agents include mannitol, sorbitol, sucrose, trehalose and amino acids like glycine, histidine, and arginine.

Bulking agents may be crystalline (such as glycine, mannitol, or sodium chloride) or amorphous (such as dextran, hydroxyethyl starch) and are generally used in formulations in an amount from 0.5% to 10%.

As shown in the examples, the sugar mannitol forms the basis of the formulation which provides long term stability to Imatinib. According to one embodiment, the bulking agent is selected from the sugars mannitol, sorbitol, sucrose, and trehalose. According to one embodiment, the bulking agent is mannitol.

Mannitol is also known as D-mannitol. It is a hexahydric alcohol related to mannose and is isomeric with sorbitol. It has a CAS registry number of 69-65-8 and has a molecular weight of 182.17. Mannitol is widely used in pharmaceutical formulations. It is generally inert and once freeze dried, rehydrates rapidly.

A buffering agent as used herein is preferably non-toxic, inert and physiologically harmless substance. The buffering agent is added to a liquid formulation to adjust and/or stabilize its pH value. Suitable buffers are well known in the art and can be found in the literature. Preferred pharmaceutically acceptable buffers comprise but are not limited to histidine-buffers, citrate-buffers, succinate-buffers, acetate-buffers and phosphate-buffers. According to one embodiment, the buffering agent is potassium dihydrogen phosphate.

According to one embodiment, the pharmaceutical composition further comprises an antioxidant. Antioxidants are used to prevent/minimize the oxidation reaction of the API or excipients over the shelf life of the product. Typical antioxidants are selected from L-methionine, sodium metabisulphite, ascorbic acid, acetylcysteine, sulfurous acid salts (bisulfite, metabisulfite), monothioglyercol, citric acid or mixtures thereof. As shown in the examples, the use of L-methionine protects the imatinib against oxidative stress. According to one embodiment, the antioxidant, is L-methionine. According to an alternative embodiment, the pharmaceutical composition does not comprise an antioxidant.

According to one embodiment, the composition is in liquid form and comprises a solvent. The term "solvent" as used herein refers to a liquid or mixture of liquids that is suitable for administration to a subject as part of a pharmaceutical composition or formulation. The solvent may be for example, selected from water, dioxolanes, dimethylacetamide, butylene glycol, polyethylene glycol, glycerin, and ethanol. In one embodiment, the solvent is water. In one embodiment the solvent is water for injection. According to one embodiment, the solvent comprises a combination of water and one more water miscible solvents selected from dioxolanes, dimethylacetamide, butylene glycol, polyethylene glycol, glycerin, ethanol, and the like, or a combination thereof. In another embodiment, the solvent is a combination of water and one additional pharmaceutically acceptable water miscible solvent. In another embodiment, the solvent is a combination of water and ethanol.

It is shown in the examples that a pH above 6.0 leads to a reduction of the solubility of the Imatinib. Therefore, the pH should be below 6.0. Preferably, the pH is in the range of 4.0 to 6.0. More preferably, the pH is in the range of 5.0 to 6.0.

According to one embodiment, the Imatinib is present in the form of Imatinib mesylate. According to one embodiment of the pharmaceutical composition, the concentration of Imatinib is in the range of 80 mg/ml to 200 mg/ml. The concentration may be for example 80 mg/ml, 90 mg/ml, 100 mg/ml, 110 mg/ml, 120 mg/ml, 130 mg/ml, 140 mg/ml, 150 mg/ml, 160 mg/ml, 170 mg/ml, 180 mg/ml, 190 mg/ml, or 200 mg/ml. According to one embodiment, the concentration of Imatinib is in the range of 100 mg/ml to 180 mg/ml. According to one embodiment, the concentration of Imatinib is in the range of 110 mg/ml to 150 mg/ml. According to one embodiment, the concentration of Imatinib is in the range of 120 mg/ml to 140 mg/ml.

The inventors defined an optimal composition of the formulation. According to one embodiment, of the pharmaceutical composition, the weight ratio of the bulking agent to Imatinib is in the range of 1:4 to 1:30. Less bulking agent than in a ratio of 1:30 may not display the structuring and stabilizing properties. More bulking agent than a ratio of 1:4 may have a negative impact on the viscosity of the product. According to one embodiment, of the pharmaceutical composition, the weight ratio of the bulking agent to Imatinib is in the range of 1:8 to 1:24, According to one embodiment, the weight ratio of the bulking agent to Imatinib is in the range of 1:12 to 1:20. According to one embodiment, the weight ratio of the bulking agent to Imatinib is in the range of 1:14 to 1:18.

Accordingly, the concentration of the bulking agent should be in the range of 2 mg/ml to 20 mg/ml. In one embodiment, the concentration of the bulking agent is in the range of 3 mg/ml to 14 mg/ml. In one embodiment, the concentration of the bulking agent is in the range of 5 mg/ml to 11 mg/ml. In one embodiment, the concentration of the bulking agent is in the range of 7 mg/ml to 9 mg/ml.

According to one embodiment of the pharmaceutical composition, the weight ratio of the antioxidant to Imatinib is in the range of 1:2 to 1:25. According to one embodiment, the weight ratio of the antioxidant to Imatinib is in the range of 1:4 to 1:20. A concentration of antioxidant below 1:25 is not expected to sufficiently protect Imatinib against oxidation. A concentration of antioxidant above 1:2 does not further improve the protection against oxidative stress. According to one embodiment, the weight ratio of the antioxidant to Imatinib is in the range of 1:8 to 1:16. According to one embodiment, the weight ratio of the antioxidant to Imatinib is in the range of 1:9 to 1:13.

Accordingly, the concentration of the antioxidant should be in the range of 4 mg/ml to 24 mg/ml. In one embodiment, the concentration of the antioxidant is in the range of 6 mg/ml to 20 mg/ml. In one embodiment, the concentration of the antioxidant is in the range of 8 mg/ml to 16 mg/ml. In one embodiment, the concentration of the antioxidant is in the range of 11 mg/ml to 14 mg/ml.

According to one embodiment of the pharmaceutical composition, the weight ratio of the buffering agent to Imatinib is in the range of 1:3 to 1:25. According to one embodiment, the weight ratio of the buffering agent to Imatinib is in the range of 1:5 to 1:20. A concentration of buffering agent below 1:25 does not sufficiently buffer the solution. A concentration of buffering agent above 1:2 does not further improve the buffering capacity. According to one embodiment, the weight ratio of the buffering agent to Imatinib is in the range of 1:9 to 1:17. According to one embodiment, the weight ratio of the buffering agent to Imatinib is in the range of 1:11 to 1:15.

Accordingly, the concentration of the buffering agent should be in the range of 2 mg/ml to 20 mg/ml, In one embodiment, the concentration of the buffering agent is in the range of 4 mg/ml to 16 mg/ml. In one embodiment, the concentration of the buffering agent is in the range of 6 mg/ml to 14 mg/ml. In one embodiment, the concentration of the buffering agent is in the range of the range of 8 mg/ml to 11 mg/ml.

According to one embodiment, the composition contains a total volume in the range of 0.5 to 10 ml. According to one embodiment, the composition contains a total volume in the range of 1 to 8 ml. According to one embodiment, the composition contains a total volume in the range of 1 to 5 ml. According to one embodiment, the composition contains a total volume in the range of 2 to 4 ml. According to one embodiment, the composition contains a total volume in the range of about 3 ml.

According to one embodiment the pharmaceutical composition comprises Imatinib in a concentration of 133.3 mg/ml, L-Methionine in a concentration of 12.5 mg/ml, mannitol in a concentration of 8.3 mg/ml, and Potassium dihydrogen phosphate in a concentration of 9.6 mg/ml. Preferably in this embodiment, the composition contains a total volume of 3 ml and the solvent is water, in particular water for injection.

The pharmaceutical composition according to the invention is of high purity and stability. As shown in the examples there are only minor impurities. In particular, the concentration of Impurity F is below 40 ppm. According to one embodiment, the concentration of Impurity F is below 20 ppm. In one embodiment, the concentration of Impurity F is below 10 ppm. In one embodiment, the concentration of Impurity F is below 5 ppm.

Moreover, the concentration of Impurity J in the pharmaceutical composition is below 0.5 % based on the concentration of Imatinib. According to one embodiment, the concentration of Impurity J is below 0.2 % based on the concentration of Imatinib. In one embodiment, the concentration of Impurity J is below 0.1 % based on the concentration of Imatinib.

According to one embodiment, the concentration of Imatinib N-oxide in the pharmaceutical composition is below 0.5 % based on the concentration of Imatinib. According to one embodiment, the concentration of Imatinib N-oxide is below 0.2 % based on the concentration of Imatinib. In one embodiment, the concentration of Imatinib N-oxide is below 0.1 % based on the concentration of Imatinib.

According to one embodiment, the concentration of Imatinib di-N-oxide in the pharmaceutical composition is below 0.5 % based on the concentration of Imatinib. According to one embodiment, the concentration of Imatinib di-N-oxide is below 0.2 % based on the concentration of Imatinib. In one embodiment, the concentration of Imatinib di-N-oxide is below 0.1 % based on the concentration of Imatinib.

Moreover, the concentration of any single unspecified impurity in the pharmaceutical composition is below 0.2 % based on the concentration of Imatinib. In one embodiment, the concentration of any single unspecified impurity is below 0.1 % based on the concentration of Imatinib.

Accordingly, the sum of impurities in the pharmaceutical has a concentration below 2.0 % based on the concentration of Imatinib. In one embodiment, the sum of impurities in the pharmaceutical has a concentration below 1.0 % based on the concentration of Imatinib. In one embodiment, the sum of impurities in the pharmaceutical has a concentration below 0.5 % based on the concentration of Imatinib. In one embodiment, the sum of impurities in the pharmaceutical has a concentration below 0.2 % based on the concentration of Imatinib.

A measure for the stability is the occurrence of impurities, such as Impurity F Impurity J, Impurity C, Imatinib di-N-oxide or Imatinib N-oxide, over the course of time. According to one embodiment, the concentration of Impurity F, Impurity J, Impurity C, Imatinib di-N-oxide or Imatinib N-oxide, any unspecified impurity and/or the sum of impurities is/are still as defined above when measured after storage for 24 h at 25 °C.

According to one embodiment, the pharmaceutical composition is lyophilized. The terms "lyophilization," "lyophilizing," and "lyophilized" as used herein refer to a freeze-drying process by which the Imatinib formulation is frozen and, while still in the frozen state, water and other solvents, if present, are removed by sublimation under vacuum. The term "lyophilate" as used herein refers to a powder obtained by lyophilization.

The lyophilized powder may be portioned, for example into individual vials. Each portion of the lyophilized powder may have a total mass of Imatinib of in the range of 200 mg to 1000 mg. The total mass of Imatinib in each portion may be 200 mg, 300 mg, 400 mg, 500 mg, 600 mg, 700 mg, 800 mg, 900 mg or 1000 mg. The total mass of Imatinib in each portion may in the range of 300 mg to 500 mg, preferably in the range of 350 to 450 mg, more preferably about 400 mg. Alternatively, The total mass of Imatinib in each portion may in the range of 700 mg to 900 mg, preferably in the range of 750 to 850 mg, more preferably about 800 mg.

Due to the formulation according to the invention, the pharmaceutical composition forms a well-formed lyophilization cake. Moreover, with lyophilization, the pharmaceutical composition becomes even more stable. Accordingly, the concentration of Impurity F, Impurity J, Impurity C, Imatinib di-N-oxide, Imatinib N-oxide, any unspecified impurity and/or the sum of impurities is/are still below the thresholds as defined above when measured after storage with a temperature of about 25 °C and/or a relative humidity of about 60 % for at least 2 months, preferably at least 4 months, more preferably at least 6 months. The concentration of Impurity J, Impurity F, Impurity J, Impurity C, Imatinib di-N-oxide, Imatinib N-oxide, any unspecified impurity and/or the sum of impurities is/are still as below the thresholds defined above when measured even at storage conditions with a temperature of about 40 °C and/or a relative humidity of about 75 % for at least 2 months, preferably at least 4 months, more preferably at least 6 months.

The lyophilization cake has a light yellow color. It maintains an appearance of light yellow or brownish yellow or greenish yellow colored cake or powderat storage conditions with a temperature of about 25 °C and/or a relative humidity of about 60 % for at least 2 months, preferably at least 4 months, more preferably at least 6 months. The lyophilization cake maintains this appearance even at storage conditions with a temperature of about 40 °C and/or a relative humidity of about 75 % for at least 2 months, preferably at least 4 months, more preferably at least 6 months.

According to one embodiment, the pH after reconstitution of the lyophilization cake is in the range of 5.0 to 7.0. The pH after reconstitution of the lyophilization cake is maintained after storage at a temperature of about 25 °C and/or a relative humidity of about 60 % for at least 2 months, preferably at least 4 months, more preferably at least 6 months. The pH is maintained even at storage at a temperature of about 40 °C and/or a relative humidity of about 75 % for at least 2 months, preferably at least 4 months, more preferably at least 6 months.

According to one embodiment, the reconstitution time of the lyophilization cake is below 5 min, preferably below 3 min, more preferably below 2 min. A freshly produced lyophilization cake most preferably has a reconstitution time below 1 min. After storage at a temperature of about 25 °C and/or a relative humidity of about 60 % for at least 2 months, preferably at least 4 months, more preferably at least 6 months, the reconstitution time of the lyophilization cake is below 5 min, preferably below 3 min, more preferably below 2 min.

The formulations of the present invention are particularly useful for the treatment of stroke, in particular hemorrhagic stroke or ischemic stroke, spinal cord injury (SCI), and traumatic brain injury (TBC).

According to one embodiment, the pharmaceutical composition is for use in the treatment of stroke, spinal cord injury (SCI), and traumatic brain injury (TBC). According to one embodiment, the treatment comprises a parenteral administration of a ready-to-use composition based on the pharmaceutical composition, wherein the parenteral administration is preferably an intravenous administration.

### Ready-to-use solution

In a second aspect, the invention relates to a ready-to-use solution for use in the treatment of stroke, which is reconstituted from a lyophilized pharmaceutical composition according to the first aspect and contains an aqueous diluent.

According to one embodiment, the ready-to-use solution pharmaceutical is for use in the treatment of stroke, in particular hemorrhagic stroke or ischemic stroke, spinal cord injury (SCI), and traumatic brain injury (TBI).

To prepare a concentrate of the ready-to-use solution, the lyophilized composition may be reconstituted, for example with water for injection.

According to one embodiment, the volume of the reconstituted concentrated is 0.5-fold to 3-fold the volume of the solvent of the pharmaceutical composition. According to one embodiment, the volume of the reconstituted concentrated solution is 1-fold to 2.5-fold the volume of the solvent of the pharmaceutical composition. According to one embodiment, the volume of the reconstituted concentrated solution is 1.2-fold to 2-fold the volume of the solvent of the pharmaceutical composition. According to one embodiment, the volume of the reconstituted concentrated solution is about 1.6-fold the volume of the solvent of the pharmaceutical composition.

This concentrate may be diluted with an aqueous diluent to a predefined concertation of Imatinib. As an example, to prepare the ready-to-use infusion solution, the concentrated solution may be withdrawn from the vial, for example using a plastic syringe and transferred into the infusion container containing the diluent.

According to one embodiment, the aqueous diluent is selected from 0.9 % saline, 0.45 % saline, Ringer's lactate and Ringer's acetate.

200 mL of 0.9% Sodium chloride solution for infusion (providing Imatinib 2.0 mg/mL diluted solution).

According to one embodiment, the volume of the reconstituted solution diluted with a factor in the range of 5 to 100, preferably in the range of 10, more preferably in the range of 20 to 60, most preferably in the range of 30 to 50. For example, a reconstituted concentrated solution with a volume of 5 ml is diluted in to a final volume of 200 ml with the aqueous diluent.

According to one embodiment of the ready-to-use solution, the concentration of Imatinib is in the range of 0.1 mg/ml to 10 mg/ml. The concentration may be, for example, 0.1 mg/ml, 0.2 mg/ml, 0.5mg/ml, 0.8 mg/ml, 1.0 mg/ml, 1.2 mg/ml, 1.5 mg/ml, 1.8 mg/ml, 2.0 mg/ml, 2.2 mg/ml, 2.5 mg/ml, 2.8 mg/ml, 3.0 mg/ml, 3.5 mg/ml, 4.0 mg/ml, 4.5 mg/ml, 5.0 mg/ml, 5.5 mg/ml, 6.0 mg/ml, 6.5 mg/ml, 7.0 mg/ml, 7.5 mg/ml, 8.0 mg/ml, 8.5 mg/ml, 9.0 mg/ml, 9.5 mg/ml, 10.0 mg/ml. According to one embodiment, the concentration of Imatinib is in the range of 0.5 mg/ml to 6.0 mg/ml. According to one embodiment, the concentration of Imatinib is in the range of 1.0 mg/ml to 3.0 mg/ml. According to one embodiment, the concentration of Imatinib is about 2.0 mg/ml.

According to one embodiment, the pH of the ready-to-use solution is in the range of 5.0 to 7.0.

According to one embodiment the treatment comprises a parenteral administration of a ready-to-use composition based on the pharmaceutical composition, wherein the parenteral administration is preferably an intravenous administration.

The ready-to-use solution facilitates safe and effective administration of Imatinib to a patient. It is particle-free, and does not form a gel or suspension. It is a clear, aqueous solution is preferred.

A clear solution is defined as a visually clear solution essentially free from any visible particulates that can be observed on a visual inspection. Generally, if any particulate matter is observed, the formulation is not suitable for intravenous administration and should not be utilised as occlusion of blood vessels may occur. Accordingly, in view of the qualitative nature of the visual test, the term "essentially free from any visible particulates" is usually applied when no visible particulate matter is observed.

According to an embodiment said ready-to-use solution is administered to a patient at a dose of Imatinib at least 400 mg/day. According to an embodiment said ready-to-use solution is administered to a patient at a dose of Imatinib at least 500 mg/day. According to an embodiment said ready-to-use solution is administered to a patient at a dose of Imatinib at least 600 mg/day. According to an embodiment said ready-to-use solution is administered to a patient at a dose of Imatinib at least 700 mg/day. According to an embodiment said ready-to-use solution is administered to a patient at a dose of Imatinib at least 800 mg/day. According to one embodiment, the ready-to-use solution administered twice a day. According to one embodiment, in the treatment of stroke, the ready-to-use solution is administered for at least four days. According to one embodiment, in the treatment of stroke, the ready-to-use solution is administered for at least five days. According to one embodiment, in the treatment of stroke, the ready-to-use solution is administered for at least 6 days.

According to one embodiment, in the treatment of TBI or SCI, the ready-to-use solution is administered for at least 10 days. According to one embodiment, in the treatment of TBI or SCI, the ready-to-use solution is administered for at least 10 days. According to one embodiment, in the treatment of TBI or SCI, the ready-to-use solution is administered for at least 12 days. According to one embodiment, in the treatment of TBI or SCI, the ready-to-use solution is administered for at least 14 days.

According to yet another embodiment, said ready to use solution is administered to a patient prior to, concurrent with, or subsequent to thrombectomy. The term "thrombectomy" is here used to define any surgical and/or mechanical removal or breakdown of a clot. In the United States, three classes of mechanical thrombectomy devices have been cleared by the FDA; coil retrievers, aspiration devices, and stent retrievers. Other devices and methods, currently under development, are also included in the definition of thrombectomy used herein.

According to yet another embodiment, said the ready-to-use solution is administered to a patient prior to, concurrent with, or subsequent to thrombolysis. The term "thrombolysis" is used herein to define any pharmacological breakdown of a blood clot. The currently most commonly used drug for thrombolytic therapy is tissue plasminogen activator (tPA), but there are alternative drugs that achieve the same effect.

Thrombolysis may involve the injection of a thrombolytic drug through an intravenous (IV) line or through a long catheter that delivers the drug directly to the site of the blockage. The chance of surviving and recovering from acute ischemic stroke is significantly improved if the patient is administered a thrombolytic drug within 12 hours after the stroke. Ideally, thrombolytic treatment should be given within the first 30 minutes after arrival at the hospital.

Most preferably the ready-to-use solution is administered to a patient as soon as possible, even before it has been determined if the patient has suffered an acute ischemic stroke or a hemorrhagic stroke.

Both thrombectomy and thrombolysis are emergency treatments, and should be given as soon as possible. For practical reasons, thrombolytic treatment is usually initiated before thrombectomy can be performed, the steps of any method disclosed herein do not have to be performed in the exact order disclosed, unless explicitly stated.

According to a preferred embodiment, freely combinable with the above, said Imatinib is administered to a patient prior to, undergoing or having undergone thrombectomy and thrombolysis. More preferably Imatinib is administered to a patient as soon as possible, even before it has been determined if the patient has suffered an acute ischemic stroke or a hemorrhagic stroke. An advantage of this is that the administration of Imatinib as such improves the outcome for the patient, and additionally makes it possible to start thrombolysis therapy later, also outside the recommended window, in cases where there has been delays for example in the patient reaching the hospital, or delays in making a correct diagnosis, or a delay in determining that the patient has suffered from an ischemic stroke and not from a hemorrhagic stroke, for example due to delays in obtaining access to CT or MRI equipment.

According to yet another embodiment, said thrombolysis comprises the administration of a tissue plasminogen activator (tPA), preferably chosen from alteplase (ACTIVASE^{®}), reteplase (RETEVASE^{®}, RAPILYSIN^{®}) and tenecteplase (TNKase^{®}). While the present disclosure focuses on the above mentioned tPA drugs, other drugs currently under development and pending approval can also be useful in a combination treatment.

According to one embodiment, freely combinable with the above embodiments, said Imatinib is administered to a patient at a starting dose of 1000 mg/day or higher on day 1, and at a dose in the interval of 650 mg/day to 1000 mg/day on the subsequent days. While the starting dose should be higher that the subsequent dose, it is currently contemplated that Imatinib should not be given at a higher dose than 1600 mg/day as the starting dose.

Further, it is contemplated that Imatinib should not be given at doses higher than 1200 mg/day on any of the consecutive days. Again, the exact dose can be adjusted by the treating physician, and adapted to the severity of the condition, the age and sex of the patient, body weight and other particulars of the patient.

It is also contemplated that at least a portion of the starting dose of Imatinib is administered in form of the ready-to use solution intravenously, whereas the subsequent, consecutive doses may be administered orally.

### Method of production

In a third aspect the invention provides a method for preparing a pharmaceutical composition according the first aspect comprising the steps of:
i) providing a first container with a first part of the solvent,
ii) adding to the first part of the solvent the buffering agent and the bulking agent and mixing to form an intermediate solution;
iii) adding the Imatinib to the first container and mixing the Imatinib with the intermediate solution to obtain a homogenous Imatinib suspension;
iv) providing a second container with a second part of the solvent;
v) adding the antioxidant to the second part of the solvent in the second container, mixing and heating the mixture to obtain an antioxidant solution;
vi) adding the antioxidant solution to the Imatinib suspension, optionally adding further solvent, and mixing to form the pharmaceutical composition; and
vii) optionally adjusting the pH.

As shown in the examples. NaOH has a negative impact on the solubility of the Imatinib. Thus, during the method of preparation, preferably no NaOH is added to the solutions. Moreover, in any intermediate composition containing the Imatinib, the pH is maintained in a range between 5.0 and 6.0. As shown in the examples, at a higher pH, the solubility of the Imatinib is reduced.

In the end of the procedure, the pH is adjusted if outside the range of 5.0 to 6.0 to a value in the range of 5.0 to 6.0.

The first part of the solvent in step ii) may be 40 % to 90 % of the volume. The second part of the solvent in step iv) may 10 % to 60 % of the volume.

According to one embodiment, the mixture of step v) is heated to a temperature in the range of 15 to 50 °C during mixing. In one embodiment, the temperature is in the range of 24 to 34 °C during mixing. In one embodiment, the temperature is in the range of 28 to 30 °C during mixing.

According to one embodiment, the antioxidant is L-methionine, the bulking agent is mannitol and the buffering agent is potassium dihydrogen phosphate and/or the solvent is WFI.

According to one embodiment, the mixing in steps ii) and v) is performed by stirring with a speed in the range of 150 to 350 rpm. According to one embodiment, it is performed with a speed in the range of 200 rpm to 300 rpm. According to one embodiment, it is performed with a speed in the range of 225 to 275 rpm.

### EXAMPLES

### Example 1 - Manufacturing

### Manufacturing Route

**Starting materials**

| **Active substance** | **Quality** |
|---|---|
| Imatinib mesilate | Ph. Eur.cur.ed. |

| **Excipients and materials** | **Quality** |
|---|---|
| Mannitol | Ph. Eur.cur.ed. |
| L-Methionine | Ph. Eur.cur.ed. |
| Potassium dihydrogen phosphate | Ph. Eur.cur.ed. |
| Water for injections | Ph. Eur.cur.ed. |
| Nitrogen, low oxygen | Ph. Eur.cur.ed. |

### Manufacturing process description:

Manufacturing of Imatinib 400 mg powder involves the following manufacturing steps:
- weighing of components;
- preparation of intermediate product;
- sterilizing filtration of intermediate product;
- aseptic filling into vials and semi-stoppering of vials;
- freeze-drying;
- stoppering of vials under reduced pressure of nitrogen atmosphere;
- sealing of vials with flip-off caps.

### Weighing of components

All starting materials were weighed before solution preparation.

### Preparation of pharmaceutical composition

The requested amount of WFI was charged into a reactor.

The pre-weighed amounts of mannitol and potassium dihydrogen phosphate and followed by API transfer to reactor L-Methionine pre-mixed solution and remaining requested WFI to 100% amount transfer.

### Sterilizing filtration of the pharmaceutical composition

The prepared solution was filtered through two filters in series - one sterilizing-grade filter and one police filter. Both filters in the filtration system were sterilized before use.

### Aseptic filling into vials and semi-stoppering of vials

Filling into vials and semi-stoppering with rubber stoppers was performed with automatic equipment. The filling equipment automatically performs recalibration of the fill dose and, if necessary, rejects vials with incorrect fill weight or incorrectly placed or missing stopper.

### Freeze-drying

The freeze-drying was performed according to the respective program:
- Minimum freezing set temperature: less than -35 °C.
- Primary drying set temperature: in range between -10 °C and -30 °C, vacuum less than 0.3 mbar.
- Secondary drying set temperature: in range between 20 °C and 40 °C, vacuum less than 0.3 mbar.
- Total duration of the freeze-drying process was (in range between 100 h to 150 h. After the freeze-drying process was completed, vacuum was relieved with sterile filtrated nitrogen (low-oxygen). Complete stoppering was done in the freeze dryer.

### Sealing of vials with flip-off caps

Sealing was performed on automatic sealing equipment using aluminum flip-off caps.

### Example 2 - Technology transfer from lab scale to manufacturing

Imatinib 133.33 mg/mL solution preparation process description for scale-up batch production is outlined below:
- 900 mL of WFI (≈50% of total requested amount of WFI) were transferred into 3.5 L bottle.
- 590 mL of WFI (≈30% of total requested amount of WFI) and pre-weighed amount of L-Methionine were transferred into 1 L bottle. Suspension was mixed with 250 rpm stirring speed (using PTFE crescent stirrer blade) and heated (heater set temperature was 50 °C) to 30 °C temperature for 26 min (L-Methionine dissolved after 14 min). After that solution was removed from a heater plate.
- Mannitol and Potassium dihydrogen phosphate were transferred into 3.5 L bottle with 900 mL of WFI. Suspension was mixed with 250 rpm stirring speed (using PTFE crescent stirrer blade) for 4 min (Mannitol and Potassium dihydrogen phosphate dissolved after 3 min).
- API was transferred into 3.5 L bottle with Mannitol and Potassium dihydrogen phosphate solution. Suspension was mixed with 300 rpm stirring speed (using PTFE crescent stirrer blade) for 9 min - homogeneous suspension was obtained.
- L-Methionine solution and remaining requested WFI to 100% amount were transferred into 3.5 L bottle with suspension. Suspension was mixed additionally with 300 rpm stirring speed (using PTFE crescent stirrer blade) for 13 min (API dissolved after 9 min after additional mixing). Suspension temperature after 3 min of mixing was 24.8 °C. pH of solution was equal to 5.3.
- Samples of Imatinib 133.33 mg/mL solution was collected and tested according to quality specifications.
- Before Imatinib 133.33 mg/mL solution filtration 1st Millipore Millipak 100 filter unit, in filtration SUS was wetted with cooled WFI with filtration speed 0.5 L/min for NLT 5 min in order to test its integrity.
- Imatinib 133.33 mg/mL solution was filtered using peristaltic pump with 150 rpm speed through the filtration SUS, consistent of one sterilizing-grade filter and one police filter with 0.22 µm membranes in series, in order to test performance of filtering system and sterilize intermediate products.

Simultaneously filter flush volume prior to Imatinib 133.33 mg/mL solution filtration was verified.
- Automatic filling of intermediate products was performed.
- Filled and semi-stoppered vials were inserted in freeze-dryer. Freeze-drying of Imatinib 133.33 mg/mL was performed.
- After completion of freeze-drying process vacuum relief with nitrogen, low oxygen, until slightly reduced nitrogen atmosphere pressure - 880 mbar (instead of 800±50 mbar due to manual control over stoppering process instead of automatic) - and stoppering of vials under this pressure (set hydraulic shelf squeezing pressure - 45 bar) was performed. Hydrophobic filter Sartofluor GA, Cat. No. 5182507T1 GA (0.2 µm) was used as sterile gas filter. After stoppering was completed, pressure in the freeze-dryer was increased to 990 mbar.
- Sealing of vials with 20 mm Flip-off caps for 456 vials Imatinib 400 mg powder vials.
- Imatinib 400 mg powder samples collection were performed; analysis were performed according to quality specification.

### Example 3 - Imatinib 400 mg powder formulation development

Different intermediate product formulations for Imatinib 400 mg powder, as well as different preparation procedures were studied. Initial requirements for the Imatinib 400 mg powder formulation were as follows: vial should contain Imatinib mesilate, mannitol, sodium hydroxide (NaOH) and Potassium dihydrogen phosphate (KH₂PO₄), pH after bulk product reconstitution with 5 mL of WFI should have been within 5.5-7.0 range.

Formulations and preparation procedures of intermediate product are described in Table 1 below:

**Table 1 Different Imatinib formulations**

| | | | | | | | | | |
|---|---|---|---|---|---|---|---|---|---|
| Formulation No. | Batc h No. | Component concentration in solution mg/mL | | | | | Fill volume in vial, mL | Obtained pH | |
| | | Imatinib mesilate | Mannit ol | KH₂ PO₄ | L-Methionine | NaOH | | Intermediate product | Bulk product reconstituted solution |
| | | | | | | | | | |
| 1 | 0803 20L | 106.2 mg/mL (equals to 88.9 mg/mL of Imatinib) | 5.55 | 6.43 | 8.33 | 0.89 | 4.5 | 6.36 after filtration | |
| | 1204 20L | | 5.55 | 6.43 | 8.33 | 0.89 | 4.5 | 6.88 before addition of API | |
| 2. | 0903 20L | 106.2 mg/mL (equals to 88.9 mg/mL of Imatinib) | 5.55 | 6.43 | 8.33 | - | 4.5 | 6.44 | 6.1 |
| 4. | 1904 20L | 106.2 mg/mL (equals to 88.9 mg/mL of Imatinib) | 5.55 | 6.43 | 8.33 | | 4.5 | 5.69 | |
| | 2204 20L | | | | | | | 5.57 | 5.3 |
| | 3704 20L | | | | | | | 5.43 | 5.2 |
| | 4404 20L | | | | | | | 5.47 | |
| | 4605 20L | | | | | | | 5.53 | 5.2 |
| 6. | 2104 20L | 106.2 mg/mL | 5.55 | 5.43 | | | 4.5 | 5.72 | |
| | 4104 20L | (equals to 88.9 mg/mL of Imatinib | | | | | | 5.45 | 5.2 |
| | 4504 20L | | | | | | | 5.53 | |
| 7 | 3404 20L | 159.30 mg/mL (equals to 133.33 mg/mL of Imatinib) | 8.33 | 9.65 | 12.50 | | 3.0 | 5.43 | |
| | 5105 20L | | | | | | | 5.43 | 5.20 |
| | 5305 20L | | | | | | | 5.49 | |
| 8 | 3504 20L | 136.5 mg/mL (equals to 114.29 mg/mL of Imatinib | 7.14 | 8.27 | 10.71 | | 3. 5 | 5.43 | |
| 9 | 3604 20L | 119.5 mg/mL (equals to 100.0 mg/mL | 7.25 | 7.24 | 9.38 | | 4.0 | 5.43 | |

Based on formulation development results conclusions were made as follows:
1. NaOH should not be included in formulation because during intermediate product preparation API is not dissolving if added into solution, which contains NaOH, or emulsion is formed if NaOH is added into solution, which contain API. In both cases homogenous solution could be easily formed, which is unacceptable.
2. The obtained pH of the pharmaceutical compositions, which did not contain NaOH, was in the range of 5.43-5.72 and could not be increased using base. As the pH of 1 % (m/V) Imatinib mesilate aqueous solution is in the range from 5.4 to 5.5, the solution pH after API addition was also not expected to vary within a wide range. Accordingly, the pH of the pharmaceutical composition is expected to be highly repeatable even for different API batches.
3. The pH of reconstituted bulk products was lower than the expected 5.5-7.0 range, i.e. in the range of 5.2-5.3, respectively.
4. Formulation of intermediate product Imatinib 133.33 mg/mL solution is to be used with 3.0 mL target fill volume as it significantly reduces primary drying stage duration during freeze-drying process.

Accordingly, the ingredients and concentrations of a preferred pharmaceutical composition, according to the invention, composition I is shown in Table 2.

**Table 2 - Ingredients of composition I.**

| **Component** | **Function** | **Amount per ml** | **Amount per vial** (3.0 mL, w/o overfill) | **Amount per vial** (3.18 mL, including overfill) |
|---|---|---|---|---|
| Imatinib mesilate (1) | Active pharmaceutical ingredient | 159.30 mg | 477.9 mg | 506.6 mg |
| equal to Imatinib (2) | | 133.33 mg | 400.0 mg | 424.0 mg |
| Mannitol (3) | Bulking agent | 8.33 mg | 25.0 mg | 26.5 mg |
| L-Methionine (3) | Antioxidant | 12.5 mg | 37.5 mg | 39.8 mg |
| Potassium dihydrogen phosphate | Buffering agent | 9.647 mg | 28.94 mg | 30.68 mg |
| WFI | Vehicle(4) | up to 1.0 mL | up to 3.0 mL | up to 3.18 mL |

### Example 3 - Influence of the Antioxidant on stability

In order to evaluate Imatinib oxidative degradation process, necessity of antioxidant in formulation and to identify potential degradation impurities, an oxidation study with composition I was performed - with and without antioxidant and with an intentionally added oxidizer - hydrogen peroxide to accelerate oxidative degradation. The samples were stored at 25±2 °C at a relative humidity of 60±2 %. The results of the measured impurities in the samples are summarized in Table 3 for the set up without hydrogen peroxide and Table 4 for the set up with hydrogen peroxide.

**Table 3 - Stability at non-oxidizing conditions**

| Batch No | 400420L | | | 390420L | | |
|---|---|---|---|---|---|---|
| Sample description | w/o antioxidant | | | with L-methionine | | |
| | | | | | | |

| Time | 0 h | 5 h | 24 h | 0 h | 5 h | 24 h |
|---|---|---|---|---|---|---|
| Impurity J (Ph. Eur) | <0.10% | <0.10% | <0.10% | <0.10% | <0.10% | <0.10% |
| Imatinib N-oxide | <0.10% | <0.10% | <0.10% | <0.10% | <0.10% | <0.10% |
| Imatinib diN-oxide | <0.10% | <0.10% | <0.10% | <0.10% | <0.10% | <0.10% |
| Any single unspecified impurity | <0.10% | <0.10% | <0.10% | <0.10% | <0.10% | <0.10% |
| Sum of impurities | <0.1% | <0.10% | <0.10% | <0.1% | <0.1% | <0.1% |
| | | | | | | |

As shown in Table 3, without hydrogen peroxide to accelerate oxidative degradation, the concentrations of the impurities remain below the detection limit.

**Table 4 - Stability at oxidizing conditions**

| Batch No | 400420L | 390420L | | | | |
|---|---|---|---|---|---|---|
| Sample description | w/o antioxidant | with L-methionine | | | | |
| | with 0.1% H₂O₂ | | | | | |

| Time | 0 h | 5 h | 24 h | 0 h | 5 h | 24 h |
|---|---|---|---|---|---|---|
| Impurity J | <0.10% | 0.11% | 0.40% | <0.10% | <0.10% | <0.10% |
| Imatinib N-oxide | <0.10% | <0.10% | <0.10% | <0.10% | <0.10% | <0.10% |
| Imatinib diN-oxide | <0.10% | <0.10% | <0.10% | <0.10% | <0.10% | <0.10% |
| Any single unspecified impurity | <0.10% | <0.10% | <0.10% | <0.10% | <0.10% | <0.10% |
| Sum of impurities | <0.1% | <0.1% | 0.40% | <0.1% | <0.1% | <0.1% |
| | | | | | | |

As shown in the table, under the harsh conditions with hydrogen peroxide to accelerate oxidative degradation, the concentration of impurity J increases a little bit to a total of 0.4% if no antioxidant is used. It remains below the detection limit in the set up with L-methionine. Moreover, the concentration of the other impurities remains below the detection limit with or without 0.1% H₂O₂.

### Example 4 - Long term storage stability

In order to evaluate the long-term storage stability of the lyophilized pharmaceutical composition, samples of the lyophilized composition I were stored at 40±2 °C at a relative humidity of 75±5 %. At the start and after 2 months, 4 months and 6 months, the appearance was evaluated, the reconstitution time, the pH as well as concentration of Impurity J, Imatinib N-oxide, Imatinib di-N-oxide and Impurity C was determined. The results of the measured impurities in the samples are summarized in Table 5.

**Table 5 - Long term storage results**

| Specification | Requirements | Test results | | | |
|---|---|---|---|---|---|
| | | 0 months | 2 months | 4 months | 6 months |
| Appearance | Light yellow or | Light yellow | Light | Light | Light |

| | brownish yellow or greenish yellow coloured cake or powder | cake | yellow cake | yellow cake | yellow cake |
|---|---|---|---|---|---|
| Reconstitution time | NMT 5 min | 56 s | 1 min 4 s | 1 min 29 s | 1 min 36 s |
| pH after reconstitution | 5.0 - 7.0 | 5.3 | 5.3 | 5.3 | 5.3 |
| Impurity J | 0.5 % | <0.10% | <0.10% | <0.10% | <0.10% |
| Imatinib N-oxide | 0.5 % | <0.10% | <0.10% | <0.10% | <0.10% |
| Imatinib diN-oxide | 0.5 % | <0.10% | <0.10% | <0.10% | <0.10% |
| Impurity C | 0.5 % | <0.10% | <0.10% | <0.10% | <0.10% |
| Any single unspecified impurity | 0.2 % | <0.10% | <0.10% | <0.10% | <0.10% |
| Sum of impurities | 2.0 % | <0.1% | <0.1% | <0.1% | <0.1% |

As shown in the table all impurities as well as the sum of impurities remained below the detection limit of 0.1 % relative to the concentration of imitamib, and therefore significantly below the required values. Even the sum of impurities remained below the detection limit at all times. In all samples, the appearance of the lyophilisate was a light yellow cake. Therefore, the appearance did not change over time and met the requirements at all times. At all time, points the pH after reconstitution remained at pH 5.3 and therefore within the required range. The reconstitution time increased with storage time from 56 s at the beginning to 96 s after 6 months of storage but remained well below the acceptance criterion of 5 min.

### Example 5 - Presence of Impurity F

According to information from Ph.Eur. monography it is known that Imatinib 400 mg powder might contain genotoxic impurity F. Additional tests (exposing Imatinib to certain stress conditions) were conducted to confirm expected content of genotoxic Impurity F in Imatinib 400 mg powder and justify its absence in quality specification.

Effect of temperature and atmosphere on Imatinib bulk product, as liquid dosage form, stability regarding Impurity F.

Imatinib 400 mg concentrate (batch No. 530520L, produced as a liquid dosage form) was tested for genotoxic Impurity F (Ph. Eur.) content after storage for 3 months at its intended accelerated (25±2 °C/60±5% RH) storage conditions. Concentrate was stored under air atmosphere in order to simulate worst-case production conditions. Results are presented in Table below.

From the results, it is seen that the amount of Impurity F remained low (3-5ppm) even after 3 months of storage at (40±2 °C/75±5% RH) conditions. Comparing these results and results of Impurity F of freshly prepared Imatinib 133.33 mg/mL solution (batch No. 870820L, refer to oxidative study) it is seen that difference is only approximately Δ3 ppm; both results are below method disregard limit (5 ppm). Based on this it can be concluded that increase of Impurity F in bulk product stored under nitrogen, low oxygen atmosphere is insignificant, and therefore, increase of Impurity F in Imatinib 400 mg powder during shelf-life at its intended storage conditions (25±2) °C/(60±5)% RH above requirement limit (100 ppm) is not expected and should not be controlled.

**Table 6 - Long term storage and Impurity F**

| Time of Testing | 3 Months | |
|---|---|---|
| Sample Set | 1 | 2 |
| Impurity F | 5.12 ppm | 3.22 ppm |
| | Average: 4 ppm | |
| | | |

Many modifications and other embodiments of the invention set forth herein will come to mind to the one skilled in the art to which the invention pertains having the benefit of the teachings presented in the foregoing description and the associated drawings. Therefore, it is to be understood that the invention is not to be limited to the specific embodiments disclosed and that modifications and other embodiments are intended to be included within the scope of the appended claims. Although specific terms are employed herein, they are used in a generic and descriptive sense only and not for purposes of limitation.

## Claims

1. A pharmaceutical composition comprising Imatinib and a stabilizing formulation, wherein said stabilizing formulation comprises a bulking agent and a buffering agent, wherein the pH of the pharmaceutical composition is below 6.0.

2. The pharmaceutical composition according to claim 1, wherein the composition is in liquid form and comprises a solvent wherein preferably the solvent is water.

3. The pharmaceutical composition according to claim 1 or 2, wherein the buffering agent is selected from a histidine-buffer, citrate-buffer, succinate-buffer, acetate-buffer and a phosphate-buffer, preferably the buffering agent is potassium dihydrogen phosphate.

4. The pharmaceutical composition according to any one of the preceding claims, wherein the bulking agent is selected from mannitol, sorbitol, sucrose, and trehalose, preferably, the bulking agent is mannitol.

5. The pharmaceutical composition according to any one of the preceding claims 1, further comprising an antioxidant, preferably selected from L-methionine and sodium metabisulphite.

6. The pharmaceutical composition according to any one of the preceding claims, wherein:
• the weight ratio of the bulking agent to Imatinib is in the range of 1:4 to 1:30, preferably in the range of 1:8 to 1:24, more preferably in the range of 1:12 to 1:20, most preferably in the range of 1:14 to 1:18;
• the weight ratio of the antioxidant to Imatinib is in the range of 1:2 to 1:25, preferably in the range of 1:4 to 1:20, more preferably in the range of 1:6 to 1:16, most preferably in the range of 1:8 to 1:12; and/or
• the weight ratio of the buffering agent to Imatinib is in the range of 1:3 to 1:25, preferably in the range of 1:5 to 1:20, more preferably in the range of 1:9 to 1:17, most preferably in the range of 1:11 to 1:15.

7. The pharmaceutical composition according to any of the preceding claims, wherein the concentration of Imatinib is in the range of 100 mg/ml to 160 mg/ml, preferable in the range of 110 mg/ml to 150 mg/ml, more preferably in the range of 120 mg/ml to 140 mg/ml, wherein preferably the Imatinib is present in the form of Imatinib mesylate.

8. The pharmaceutical composition according to any one of the preceding claims, wherein
• the concentration of Impurity F is below 40 ppm, preferably below 20 ppm, more preferably below 10 ppm, most preferably below 5 ppm, wherein preferably, the concentration of Impurity F is measured after storage for 24 h at about 25 °C;
• the concentration of Impurity J is below 0.5 %, preferably below 0.2 % more preferably below 0.1 % based on the concentration of Imatinib.

9. The pharmaceutical composition according to any one of the preceding claims, wherein the pharmaceutical composition is lyophilized, wherein preferably the composition comprises Imatinib in a dose of 200 mg to 600 mg, preferably 300 mg to 500 mg, more preferably 350 mg to 450 mg.

10. The pharmaceutical composition according to claim 9, wherein after storage at a temperature of about 40 °C and/or a relative humidity of about 75 % for 6 months:
• the concentration of Impurity J, Imatinib di-N-oxide, and/or Imatinib N-oxide, is below 0.5 %, preferably below 0.2 %, more preferably below 0.1 % based on the concentration of Imatinib;
• the concentration of Impurity F is below 40 ppm, preferably below 20 ppm, more preferably below 10 ppm;
• the concentration of any unspecified impurity is below 0.2 %, preferably below 0.1 % based on the concentration of Imatinib;
• the sum of impurities in the pharmaceutical has a concentration below 2.0 %, preferably below 1.0 %, more preferably below 0.5 %, most preferably below 0.2 % based on the concentration of Imatinib,
• the reconstitution time of the lyophilization cake is below 5 min, preferably below 3 min, more preferably below 2 min.

11. A ready-to-use solution for use in the treatment of stroke, spinal cord injury (SCI), traumatic brain injury (TBI) which is reconstituted from a lyophilized pharmaceutical composition according to claim 10 by the addition of an aqueous diluent.

12. The ready-to-use solution for use according to claim 10, wherein the treatment comprises a parenteral administration, wherein the parenteral administration is preferably an intravenous administration.

13. The ready-to-use solution for use according to claim 10 or 11, wherein the aqueous diluent is selected from 0.9 % saline, 0.45 % saline, Ringer's lactate and Ringer's acetate.

14. A method for preparing a pharmaceutical composition according to any one of claims 1 to 9 comprising the steps of:
i) providing a first container with a first part of the solvent, in particular 40 % to 90 % of the volume;
ii) adding to the first part of the solvent the buffering agent and the bulking agent and mixing to form an intermediate solution;
iii) adding the Imatinib to the first container and mixing the Imatinib with the intermediate solution to obtain a homogenous Imatinib suspension;
iv) providing a second container with a second part of the solvent, in particular 10 % to 50 % of the volume;
v) adding the antioxidant to the second part of the solvent in the second container, mixing and heating the mixture to obtain an antioxidant solution;
vi) adding the antioxidant solution to the Imatinib suspension, optionally adding further solvent, and mixing to form the pharmaceutical composition; and
vii) optionally adjusting the pH.

15. The method according to claim 13, wherein the mixture of step v) is heated to a temperature in the range of 15 to 50 °C, preferably 24 to 34 °C, more preferably in the range of 28 to 30 °C during mixing.

16. The method according to any of claims 13 or 14, wherein in any intermediate composition containing the Imatinib the pH is maintained in a range between 4.0 and 6.0.
